# EUROPEAN PATENT SPECIFICATION

(11) **EP 1 633 376 B1**
(45) Date of publication and mention of the grant of the patent: **16.04.2008**
(21) Application number: 04754081.0
(22) Date of filing: 01.06.2004
(51) Int. Cl.: A61K 33/30, A61P 11/00

(54) **COMPOSITIONS FOR PREVENTION AND TREATMENT OF COLD AND INFLUENZA-LIKE SYMPTOMS COMPRISING CHELATED ZINC**
ZUSAMMENSETZUNGEN ZUR PRÄVENTION UND BEHANDLUNG VON ERKÄLTUNG UND GRIPPE-ÄHNLICHEN SYMPTOMEN, DIE CHELIERTES ZINK ENTHALTEN
COMPOSITIONS DE PREVENTION ET DE TRAITEMENT DE SYMPTOME DE TYPE RHUME ET GRIPPE COMPRENANT DU ZINC CHELATE

(30) Priority: 06.06.2003 US 456465
(43) Date of publication of application: 15.03.2006
(73) Proprietor: THE PROCTER & GAMBLE COMPANY, Cincinnati, Ohio 45202 (US)
(72) Inventor: RENNIE, Paul, John, Godalming Surrey GU7 2HA (GB); DE LA HARPE, Shane, Michael, Liskeard Cornwall PL14 4BP (GB); KHANOLKAR, Jayant, Ekanth, Surbiton Surrey KT5 8RD (GB); MCDONALD, Michael, Ray, Middletown, OH 45044 (US); SUTTON, Richard, Matthew, Charles, Camberley Surrey GU15 2PB (GB)
(74) Representative: Wilding, Richard Alan
(86) International application number: PCT/US2004/017390
(87) International publication number: WO 2004/110463

(56) References cited:
- GB-A- 2 306 109
- US-A- 4 503 070
- US-A- 5 409 905
- US-A- 5 622 724
- ZAREMBO J E ET AL: "ZINC(II) IN SALIVA: DETERMINATION OF CONCENTRATIONS PRODUCED BY DIFFERENT FORMULATIONS OF ZINC GLUCONATE LOZENGES CONTAINING COMMON EXCIPIENTS" JOURNAL OF PHARMACEUTICAL SCIENCES, AMERICAN PHARMACEUTICAL ASSOCIATION. WASHINGTON, US, vol. 81, no. 2, 1 February 1992 (1992-02-01), pages 128-130, XP000612191 ISSN: 0022-3549

## Description

### FIELD OF THE INVENTION

The present invention is directed to respiratory tract compositions for the prevention and treatment of cold and influenza-like symptoms due to respiratory tract viral infections, wherein these compositions are effective in both preventing the onset of the symptoms of colds and influenza or significantly mitigating them if an individual is already afflicted with such symptoms. In particular, the present invention is directed to respiratory tract compositions, particularly nasal compositions, comprising a combination of chelated and unchelated ionic zinc compounds that provide for the prevention and treatment of cold and influenza-like symptoms.

### BACKGROUND OF THE INVENTION

It is known that many different viruses and viral strains bring on symptoms associated with respiratory viral infections including symptoms associated with the common cold and influenza. A number of remedies to alleviate the symptoms of the common cold and influenza have been suggested. Typical remedies to alleviate symptoms of the common cold include cough/cold products that contain one or more of the following actives: nasal decongestants such as pseudoephedrine, oxymetazoline, antihistamines such as doxylamine, antitussives such as dextromethorphan, expectorants such as guaifenesin and anti-pyretics such as acetaminophen. Treatment of symptoms associated with influenza includes vaccination and the use of specific antiviral drugs, including those antiviral drugs that have been reviewed by A. Elliot and J. Ellis, 2000, Pharmaceutical Journal, 265, 446-451.

In an attempt to improve existing cold remedies, experts in the field have suggested several alternative pharmacotherapies and subsequently conducted cold trials to test their efficacy. Examples of these therapies include: the use of interferon-α₂, Douglas et al., Prophylactic Efficacy of Intranasal Alpha2- Interferon Against Rhinovirus Infection in the Family Setting, The New England Journal of Medicine, 314, pp. 65-70, 1986; bradykinin antagonist, Higgins et al., A Study of the Efficacy of the Bradykinin Antagonist NPC567 in Rhinovirus Infections in Human Volunteers, Antiviral Research vol. 14, pp. 339-344, 1990; glucocorticoid, Farr et al., A Randomized Controlled Trial of Glucocorticoid Prophylaxis Against Experimental Rhinovirus Infection, Journal of Infectious Diseases, vol. 162, pp. 1173-1177, 1990; nedocromil, Barrow et al., The Effect of Intranasal Nedocromil Sodium on Viral Upper Respiratory Tract Infections in Human Volunteers, Clinical and Experimental Allergy, vol. 20, pp. 45-51, 1990; a combination of interferon-α₂, ipratropium and naproxen, Gwaltney, Combined Antiviral and Antimediator Treatment of Rhinovirus Colds, The Journal of Infectious Diseases vol. 166, pp. 776-782, 1992; zinc salts, Potter et al., DIAS_ Rounds, Zinc Lozenges for Treatment of Common Colds, The Annals of Pharmacotherapy, vol. 27, pp. 589-592, 1993.

Other disclosures of compositions and methods for the prevention and treatment of the common cold include U.S. Patents 5,240,694; 5,422,097; and 5,492,689; all to Gwaltney, which disclose treatments using combinations of anti-viral and anti-inflammatory compounds. Further disclosures include U.S. Patents Re 33,465 and 5,409,905; both to Eby disclosing treatment using zinc salts; U.S. Patent 5,626,831 to Van Moerkerken disclosing treatments using orally administered aminocarboxylic acid compounds; U.S. Patents 4,619,934 and 4,552,899, both to Sunshine, disclosing treatment of cough and colds using compositions comprising non-steroidal anti-inflammatory drugs such as NSAIDS with antihistaminically effective materials such as chlorpheniramine.

More specific disclosures of compositions and methods for the prevention and treatment of the common cold include those nasal compositions as described in U.S. Patents 4,689,223; 6,187,332; 6,080,783; 6,365,624; 5,622,724; and 4,956,385. U.S. Patents 6,080,783; 6,365,624; 5,622,724; and 4,956,385 disclose compositions and methods for the prevention and/or treatment of the common cold wherein the compositions comprise ionic zinc such that the ionic zinc contacts the nasal membrane.

U.S. Patent 5,622,724 further describes the ionic zinc as a substantially unchelated zinc ion. This disclosure states that nasal compositions, particularly nasal sprays, can provide for the treatment or prophylaxis of the common cold by incorporating a substantially unchelated ionic zinc compound as compared to chelated zinc compounds into the formulation.

It has been surprisingly found that compositions for the prevention and treatment of the common cold can be formulated to comprise a combination of chelated and unchelated zinc ions such that this combination is highly effective in treating cold symptoms, especially when the compositions are formulated to contain substantially chelated zinc ions.

Therefore, it is desirable to formulate compositions for the prevention and treatment of the common cold that can be administered nasally, and that can comprise ionic zinc with minimal or no perceived irritation that can be associated with ionic zinc. These compositions can be formulated to comprise a combination of chelated and unchelated zinc ions to provide for cold prevention and treatment efficacy that is equivalent or superior to compositions comprising either chelated or unchelated zinc ions, or even to compositions comprising substantially unchelated zinc ions.

### SUMMARY OF THE INVENTION

The present invention is directed to respiratory tract compositions and methods for using such compositions for prevention and treatment of cold and influenza-like symptoms due to respiratory tract viral infections. The respiratory tract compositions of the present invention are preferably applied to the areas of the respiratory tract such as the nasal cavity to provide for a surface pH of the nasal cavity tissue of from pH 3.0 to 5.5. These compositions create an environment hostile to viral infections through the incorporation of a combination of chelated and unchelated zinc ions into the compositions.

Preferably, the present invention is directed to nasal compositions, particularly nasal sprays, that comprise chelated and unchelated zinc ions, wherein the quantity of zinc ions is at least 50.1% chelated zinc ions. Specifically, the present invention is directed to a preferred embodiment of a respiratory tract composition such as a nasal composition comprising (a) from 0.001% to 20% by weight of an ionic zinc compound providing a quantity of from 0.1% to 49.9% of unchelated zinc ions, and (b) from 0.001% to 20% by weight of an ionic zinc compound providing a quantity of from 50.1% to 99.9% of chelated zinc ions.

The present invention is also preferably directed to a method of treating symptoms of the cold or influenza, wherein the method comprises the steps of (a) preparing a respiratory tract composition that has (i) from 0.001% to 20% by weight of an ionic zinc compound providing a quantity of from 0.1% to 49.9% of unchelated zinc ions, and (ii) from 0.001% to 20% by weight of an ionic zinc compound providing a quantity of from 50.1% to 99.9% of chelated zinc ions; and (b) administering the respiratory tract composition to a respiratory area for contact of the composition with mucosal tissue and fluid. The respiratory tract compositions are especially effective when the compositions are administered using nasal sprays.

It has been found that upon application to the nasal tissues, the respiratory tract compositions of the present invention create an environment hostile to viruses. Such an environment deters viruses from infecting the nasal cavity which is subsistent to respiratory tract viral infections. The respiratory tract compositions of the present invention are also suitable for treating already infected subjects in order to mitigate cold and influenza-like symptoms, as well as being suitable for method applications of reducing or eliminating the possibility of acquisition of such viruses when confronted with a high-risk public environment including schools and office buildings.

It has also been found that the respiratory tract compositions of the present invention are non-irritating even though the compositions contain ionic zinc. It is believed that the ionic zinc provides for an environment that is hostile to viruses to result in the prevention of a viral infection or in the reduction of the severity or duration of a viral infection. The respiratory tract compositions defined herein comprise a combination of chelated and unchelated ionic zinc to prevent and treat symptoms of the common cold or influenza, and it has been found that this combination of chelated and unchelated zinc ions provides for effective prevention and treatment of cold or influenza-like symptoms at a quantity of chelated ionic zinc of at least 50.1%.

### DETAILED DESCRIPTION OF THE INVENTION

The compositions of the present invention are respiratory tract compositions that are effective in the prevention and treatment of cold and influenza-like symptoms. These compositions are especially effective in the prevention and treatment of cold and influenza like symptoms when the compositions are administered in the form of nasal compositions. The respiratory tract compositions of the present invention are typically administered using a pharmaceutically acceptable vehicle sometimes referred to as carrier systems.

The term "respiratory tract compositions" as used herein refers to compositions in a form that is directly deliverable to the airway passages from the nose and mouth. These compositions include, but are not limited to, droppers, pump sprayers, irrigation devices, pressurized sprayers, atomizers, air inhalation devices and other packaging and equipment known or yet to be developed.

The respiratory tract compositions are administered to prevent and treat "cold and influenza-like symptoms". As used herein "cold and influenza-like symptoms" refer to symptoms typically associated with respiratory tract viral infections. These symptoms include, but are not limited to, nasal congestion, chest congestion, sneezing, rhinorrhea, fatigue or malaise, coughing, fever, chills, body ache, sore throat, headache, and other known cold and influenza-like symptoms.

The term "respiratory viruses" as used herein refers to those viruses that are causal agents of cold and influenza-like symptoms. These viruses include Rhinovirus, Myxovirus (Influenza virus), Paramyxovirus (Parainfluenza virus), Respiratory Syncytial virus, Adenovirus and Coronavirus.

The term "pharmaceutically acceptable vehicle" refers to any solid, liquid or gas combined with components of the respiratory tract compositions of the present invention to deliver the components to the respiratory tract of the user. These vehicles are generally regarded as safe for use in humans, and are also known as carrier systems.

The term "chelated" as used herein refers to the complexation of a zinc ion including the binding, or partial binding, of a zinc ion with ligands, wherein the zinc ion has at least two sites of attachment to a single ligand molecule. In the alternative, the term "unchelated" refers to the free dissociation of zinc ions, or complexation of a zinc ion with ligands wherein the zinc ion has one site of attachment to a single ligand molecule.

The respiratory tract compositions of the present invention can comprise, consist of, or consist essentially of the elements and limitations of the invention described herein, as well as any of the additional or optional ingredients, components, or limitations described herein.

All percentages, parts and ratios are by weight of the respiratory tract compositions, unless otherwise specified. All such weights as they pertain to listed ingredients are based on the Specific ingredient level and, therefore, do not include carriers or by-products that may be included in commercially available materials, unless otherwise specified.

### Ionic Zinc Compound

The respiratory tract compositions of the present invention comprise a safe and effective amount of an ionic zinc compound. Ionic zinc compounds are commonly referred to as "metal salts", wherein the zinc ion substituent is believed to be the primary component that provides for a hostile environment for cold and influenza viruses. The terms "ionic zinc compound" and "ionic zinc compounds" are used interchangeably herein to include the singular and plural context of these terms. The term " safe and effective amount" as used herein refers to an amount which provides a therapeutic benefit with minimal or no adverse reactions.

It is known that metal ions such as zinc can provide antiviral properties for the prevention and treatment of cold and influenza-like symptoms. Particularly, zinc and its possible effects on common colds has been extensively documented, The Handbook for Curing the Common Cold, George A. Eby, published 1994, George Eby Research, Texas, USA. The mechanism of its action is thought to be multifactorial. Zinc ions have been shown to be both antiviral and antibacterial. They are believed to inhibit cleavage of rhinovirus polypeptides, preventing replication and formation of infective virions. Zinc ions reduce the ability of rhinoviruses to penetrate cell membranes, partly by lowering expression of intercellular adhesion molecule ICAM. Zinc ions have also been shown to stimulate T-cell lymphocytes, including production of the natural antiviral, interferou-gamma. They stabilize cell plasma membranes, protecting cells from cytotoxic agents, and preventing cell leakage.

The ionic zinc compound preferably either provides for chelated or unchelated zinc ions in the respiratory tract compositions of the present invention. The quantity of chelated and unchelated zinc ions in compositions, particularly aqueous-based compositions, can vary dependent upon the pH of the composition. The respiratory tract compositions of the present invention are preferably aqueous-based compositions that have a pH in the range of from 3.0 to 5.5, and typically comprise chelated zinc ions of quantities ranging from 50.1% to 99.9%, preferably from 50.1% to 65%; wherein the quantity of unchelated zinc ions ranges from 0.1% to 49.9%, preferably from 35% to 49.9%.

The ionic zinc compound suitable for providing chelated zinc ions can be included in the respiratory tract compositions as an individual ionic zinc compound or as a combination of ionic zinc compounds. The total concentration of ionic zinc compound in the respiratory tract compositions typically ranges from 0.001% to 20%, preferably from 0.01% to 10%, more preferably from 0.05% to 5%, most preferably from 0.05% to 2%, by weight of the composition. Likewise, one or more ionic zinc compounds suitable for providing unchelated zinc ions can be included in the respiratory tract compositions at total concentrations ranging from 0.001% to 20%, preferably from 0,01% to 10%, more preferably from 0.05% to 5%, most preferably from 0.05% to 2%, by weight of the composition.

Suitable ionic zinc compounds that provide for chelated zinc ions include those ionic zinc compounds wherein the zinc ion is bound or partially bound with a ligand, for example a chelator ligand, at at least two sites of attachment of the ligand. Specific nonlimiting examples of such ionic zinc compounds include, but are not limited to, zinc citrate, zinc ethylenediaminetetraacetic acid disodium salt (zinc-EDTA), zinc succinate, zinc tartrate, zinc malate, and mixtures thereof.

Suitable ionic zinc compounds that provide for unchelated zinc ions include those ionic zinc compounds that allow free dissociation of zinc ions in aqueous based solutions, or that have a zinc ion attached to one site of a ligand moleule. Such ionic zinc compounds include, but are not limited to, zinc acetate, zinc sulphate, zinc chloride, zinc gluconate, zinc lactate, zinc ascorbate, and mixtures thereof.

### Zinc Ions Determination

The respiratory tract compositions of the present invention comprise chelated and unchelated zinc ions. Ionic zinc compounds are incorporated into the compositions wherein these ionic zinc compounds preferably provide for chelated or unchelated zinc ion species. It is understood, however, that an ionic zinc compound can provide for both chelated and unchelated zinc ions dependent upon factors such as the pH of the composition, solubility of the ionic zinc compound, and so forth. The respiratory tract compositions of the present invention comprise ionic zinc compounds such that at least 50.1% of zinc ions are chelated zinc ions.

The method for determining chelated and unchelated zinc ions in the respiratory tract compositions include any known or otherwise effective technique for quantifying speciation levels in aqueous based solutions. Suitable methods include the MINEQL database, Version 4.5, available from Environmental Research Software (USA); and the Stability constants database and species programs available from Academic Software such as the IUPAC Stability Constants Database, Version 5.16 (Data Version 4.22) and the Species Program, Version 3.2, both available from Academic Software located in the United Kingdom.

Typically, for determining the quantity of chelated and unchelated zinc ions in a composition such as a respiratory tract composition of the present invention, the molar concentration of all zinc species is calculated, given the total concentrations of reactants and overall stability constants of the zinc complexes considered. The stability constants, which are based on formation constants (metal-ligand equilibria formation) and protonation constants (proton-ligand equlibria), can be found in databases such as the MINEQL Database, Version 4.5; the "Critical Stability Constants" database by R.E. Smith and A.E Martell, Plenum Press, New York, 1976; and the IUPAC Stability Constants Database, Version 5.16 (Data Version 4.22) available from Academic Software; which databases are incorporated herein by reference. Then, the quantity of chelated and unchelated zinc ions are calculated for Zn²⁺ ion and each Zn-ligand species.

By way of example, below is an exemplified process for the calculation of the quantity of chelated and unchelated zinc ions in a respiratory tract compositions that has a pH of 3.5 and that comprises ionic zinc compounds such as zinc acetate and zinc-EDTA as well as optional zinc-speciation molecules such as succinic acid, pyroglutamic acid (PCA), and disodium succinate. As listed in Table 1 hereinbelow, the chemical information of the formulation materials is obtained including the calculation of the molarity based on concentration percentages.

**Table 1. Chemical Information**

| **Structure** | **Name** | **MW, g/mol** | **Molarity,* moles/L** | **Reference** |
|---|---|---|---|---|
| HO₂C-CH₂-CH₂-CO₂H | Succinic Acid | 118.09 | 0.08809 | Vol. 3 Page 108** |
| 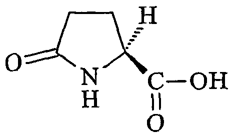 | (S)-(-)2-Pyrrolidone-5-carboxylic acid (PCA) | 129.12 | 0.02822 | Vol. 1 Page 69** |
| (CH₃CO₂)₂Zn●2H₂O | Zinc Acetate Dihydrate | 219.50 | 0.005474 | Mineql 4.5+ standard library |
| NaO₂C-CH₂-CH₂-CO₂Na_{*6H₂O} | Disodium Succinate hexahydrate | 270.05 | 0.016943 | Mineql 4.5+ standard library |
| (NaO₂CCH₂)₂NCH₂CH₂N(CH₂CO₂)Zn | Zinc-Disodium Salt of EDTA | 395.57 | 0.006707 | Mineql 4.5+ standard library |

| | | | | |
|---|---|---|---|---|
| *Molarity calculated from percentage of material in the respiratory tract composition. ** R.E. Smith and A.E. Martell Critical Stability Constants; Plenum Press: New York, 1976. | | | | |

Next, as listed in Table 2 hereinbelow, the concentration molarity of Zn(2+) and zinc-speciation molecules is determined.

**Table 2. Results for Zinc Species in Calculations:**

| | **Species** | **Concentration Molarity** |
|---|---|---|
| **Type I** | H2O | 1.00E+00 |
| | H(+) | 3.16E-04 |
| | Zn(2+) | 4.29E-03 |
| | Acetate | 5.69E-04 |
| | EDTA-4 | 8.71E-19 |
| | Succinic Acid (Succ) | 4.54E-04 |
| | Pyro-Glutamic Acid (PCA) | 3.63E-09 |
| **Type II** | OH- | 3.18E-11 |
| | Zn(OH)4-2 | 1.39E-29 |
| | Zn(OH)3- | 1.10E-20 |
| | ZnOH+ | 1.37E-08 |
| | Zn(OH)2 | 6.89E-14 |
| | ZnOH[EDTA] | 7.46E-12 |
| | ZnH[EDTA] | 2.97E-03 |
| | Zn[EDTA] | 3.74E-03 |
| | Zn[Acetate]2 | 6.11E-07 |
| | Zn[Acetate] | 9.28E-05 |
| | ZnHSucc | 9.75E-04 |
| | Zn(Succ) | 1.12E-04 |
| | Zn(Succ)2 | 8.83E-08 |
| | Zn(PCA)3 | 4.09E-16 |
| | Zn(PCA)2 | 3.18E-10 |
| | Zn(PCA) | 2.20E-06 |

Then, as listed hereinbelow in Table 3 and Figure 1, the quantity of chelated and unchelated zinc is calculated based on the concentration molarity, and stability constants using the MINEQL input and output databases.

**Table 3. Results for Species Calculations:**

| **Species** | **Concentration. Molarity** | **% of Zinc** |
|---|---|---|
| Zn(2+) | 4.29E-03 | **35.2** |
| ZnOH[EDTA] | 7.46E-12 | 0.00 |
| ZnH[EDTA] | 2.97E-03 | **24.4** |
| Zn[Acetate]2 | 6.11E-07 | 0.01 |
| Zn[Acetate] | 9.28E-05 | **0.8** |
| Zn[EDTA] | 3.74E-03 | **30.7** |
| Zn(Succ) | 1.12E-04 | **0.9** |
| ZnHSucc | 9.75E-04 | **8.0** |
| Zn(PCA)3 | 4.09E-16 | 0.00 |
| Zn(PCA)2 | 3.18E-10 | 0.00 |
| Zn(PCA) | 2.20E-06 | 0.00 |
| TOTAL | | 100.00 |

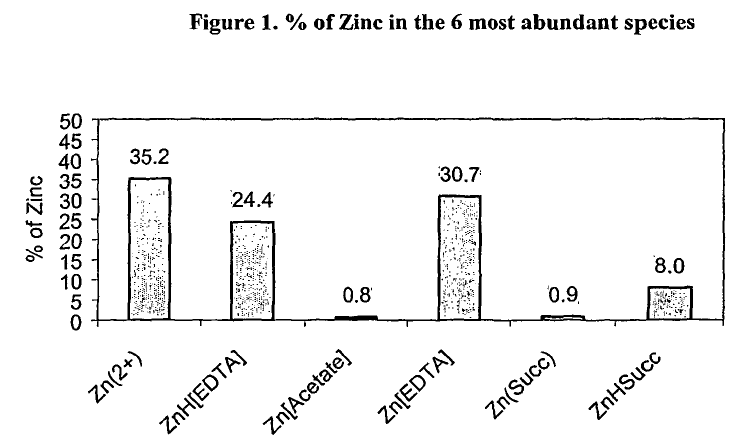

As shown by the above exemplified process, the respiratory tract composition comprises at least 50.1% of chelated zinc ions. It has been surprisingly found that respiratory tract compositions that comprise a combination of chelated and unchelated zinc ions, wherein at least 50.1% of the zinc ions are chelated, are especially effective in the prevention and treatment of symptoms associated with the common cold or influenza.

### Pharmaceutically Acceptable Vehicle

The respiratory tract compositions of the present invention are typically administered to respiratory tract areas as formulations comprising a pharmaceutically acceptable vehicle or carrier system. Any pharmaceutically acceptable vehicle in the form or a liquid, solid, or gas is suitable for the delivery of the respiratory tract compositions to prevent and treat cold and influenza-like symptoms.

Depending on the desired form and delivery device to be used, the respiratory tract compositions of the present invention can be combined with pharmaceutically acceptable vehicles such as water; water-miscible solvents including ethanol, propylene glycol, polyethylene glycol, transcutol, glycerol, and other known or otherwise effective water-miscible solvents; liquid aerosol propellants; and mixtures thereof.

When the respiratory tract compositions are administered using water as a pharmaceutically acceptable vehicle, the water is preferably purified or de-ionized water and is free of organic impurities. The concentration of water utilized to formulate the respiratory tract compositions into a final product form for delivery to respiratory tract areas ranges from 40% to 99.98%, preferably from 80% to 99.95%, by weight of the final product formulation. Respiratory tract compositions comprising water as a pharmaceutically acceptable vehicle can be analyzed with or without modifications to calculate the quantity of chelated and unchelated zinc ions present in the compositions.

When the respiratory tract compositions of the present invention are administered using a solid pharmaceutically acceptable vehicle, the vehicle may be applied in a powder form. In other words, the respiratory tract compositions of the present invention can be applied as a solid powder containing the essential ionic zinc compound and any optional components described herein with or without any known or otherwise effective solidification aids. However, pharmaceutically acceptable solid vehicles can be added to provide aid in processing of the compositions, to aid in the consistency of the compositions, to provide for improved stability, to facilitate handling, for hygroscopicity benefits, and so forth. Pharmaceutically acceptable solid vehicle materials include ingredients such as particulate and powder fillers, for example, a lactose powder. For respiratory tract compositions in the form of nasal compositions that are administered using a solid powder pharmaceutically acceptable vehicle, the particle size of the powder is typically greater than 10 microns, especially when the nasal composition is a nasal inhalant. Respiratory tract compositions comprising a solid pharmaceutically acceptable vehicle are typically dissolved or dispersed in an aqueous medium prior to the determination of the quantity of chelated and unchelated zinc ions present in the compositions.

### Optional Components

The respiratory tract compositions of the present invention may further comprise one or more optional components known or otherwise effective for use in pharmaceutical compositions, provided that the optional components are physically and chemically compatible with the ionic zinc compounds described hereinabove, or do not otherwise unduly impair product stability, aesthetics, or performance. Optional components suitable for use herein include materials such as meta! compounds other than zinc-containing metal compounds, pyroglutamic acid, organic acids, mucoadhesive polymers, pH adjusting agents, chelating agents, preservatives, sensates, sweeteners, flavours, volatile oils, mucilages, surfactant spreading aids including polyoxyethylene (20) sorbitan mono-oleate commercially sold as Polysorbate 80, and so forth, The optional components can be included in the respiratory tract compositions at concentrations ranging from 0.001% to 30%, preferably from 0.01% to 10%, by weight of the composition.

The respiratory tract compositions of the present invention can optionally comprise homeopathic ingredients. A detailed, but not necessarily a complete list, of such homeopathic ingredients is found in The Homeopathic Pharmacopoeia of the United States, 1999 ed., published by The Pharmacopoeia Convention of the American Institute of Homeopathy, ©1982, Vol. 1-4, which descriptions are incorporated herein by reference. Specific nonlimiting examples of known, homeopathic, or otherwise effective, optional components suitable for use herein are described in more detail hereinbelow.

### Optional Metal Compound

The respiratory tract compositions of the present invention optionally comprise a metal compound other than the essential ionic zinc compound defined hereinabove. Suitable optional metal compounds include those commonly referred to "metal salts" that contain for example iron, silver and copper metal ion substituents.

Suitable optional metal compounds include those metal compounds containing a metal ion selected from the group consisting of manganese (Mn), silver (Ag), tin (Sn), iron (Fe), copper (Cu), aluminum (A1), nickel (Ni), cobalt (Co), and mixtures thereof. Preferred optional metal compounds include those metal compounds which contain Cu, or Fe, metal ions, or combinations thereof.

Nonlimiting examples of an optional metal compound suitable for use herein include the metal compounds referred to as salicylates, fumarates, benzoates, glutarates, lactates, citrates, malonates, acetates, glycolates, thiosalicylates, adipates, succinates, gluconates, aspartates, glycinates, tartrates, malates, maleates, ascorbates, chlorides, sulphates, nitrate, phosphates, fluorides, iodides, pidolates, and mixtures thereof. If present, the optional metal compound is included in the respiratory tract compositions of the present invention at concentrations ranging from 0.001% to 20%, by weight of the composition.

### Optional Pyroglutamic Acid

The respiratory tract compositions of the present invention preferably comprise an optional Pyroglutamic acid. The optional pyroglutamic acid has been found to be effective in providing for a surface pH of the nasal cavity tissue of from pH 3.0 to 5.5 when used in combination with an optional organic acid having a pKa value from 3.0 to 5.5. The respiratory tract compositions of the present invention comprise the optional pyroglutamic acid at concentrations ranging from 0.01% to 20%, preferably from 0.1% to 10%, more preferably from 0.25% to 8%, most preferably from 0.1% to 5%, by weight of the composition.

The optional pyroglutamic acid suitable for use in the respiratory tract compositions of the present invention includes those pyroglutamic acid compounds collectively referred to as stereoisomers and tautomers of pyroglutamic acid, Pyroglutamic acid, which is also referred to as pyrrolidone carboxylic acid has two stereoisomers (D and L) and each are preferred for optional use herein. Pharmaceutically acceptable salts of pyroglutamic acid are also suitable for optional use herein.

The D stereoisomer of pyroglutamic acid is also known by the following names: D-Proline, 5-oxo-(+)-2-Pyrolidone-5-carboxylic acid, (+)-Pyroglutamic acid, (R)-2-Pyrrolidone-5-carboxylic acid, 5-Oxo-D-proline, D-2-Pyrrolidone-5-carboxylic acid, D-Pyroglutamic acid, D-Pyrrolidinonecarboxylic acid, and D-Pyrrolidonecarboxylic acid.

The L stereoisomer of pyroglutamic acid is also known by the following names: L-Proline, 5-oxo-(-)-2-Pyrrolidone-5-carboxylic acid, (-)-Pyroglutamic acid, (5S)-2-Oxopyrrolidine-5-carboxylic acid, (S)-(-)-2-Pyrrolidone-5-carboxylic acid, (S)-2-Pyrrolidone-5-carboxylic acid, (S)-5-Oxo-2-pyrrolidinecarboxylic acid, (S)-Pytoglutamic acid, 2-L-Pyrrolidoe-5-carboxylic acid, 2-Pyrrolidinone-5-carboxylic acid, 5-Carboxy-2-pyrrolidinone, 5-Oxo-L-proline, 5-Oxoproline, 5-Pyrrolidinone-2-carboxylic acid, Glutimic acid, Glutiminic acid, L-2-Pyrrolidone-5-carboxylic acid, L-5-Carboxy-2-pyrrolidinone, L-5-Oxo-2-pyrrolidinecarboxylic acid, L-5-Oxoproline, L-Glutamic acid, gamma-lactam, L-Glutimic acid, L-Glutiminic acid, L-Pyroglutamic acid, L-Pyrrolidinonecarboxylic acid, L-Pyrrolidonecarboxylic acid, Oxoproline, PCA, Pidolic acid, Pyroglutamic acid, Pyrrolidinonecarboxylic acid, Pyrrolidone-5-carboxylic acid, and Pyrrolidonecarboxylic acid.

The DL form of pyroglutamic acid (a mixtures of the D and L stereoisomers) is known by the following names: DL-Proline, 5-oxo-(.+-.)-2-Pyrrolidone-5-carboxylic acid, (.+-.)-Pyroglutamic acid, 5-Oxo-DL-proline, DL-2-Pyrrolidinone-5-carboxylic acid, DL-2-Pyrrolidone-5-carboxylic acid, DL-Pyroglutamate, DL-Pyroglutamic acid, DL-Pyrrolidonecarboxylic acid, and Oxoproline. The DL form is also commercially available from Ajinomoto under the tradenames Ajiderw A 100 and Ajidew N 50 (Na-PCA).

Some of the above-listed stereoisomers are commercially available from UCIB, France via Bamet Products Corp., New Jersey. Such compounds are sold under trade names like Cuivridone (Cu-PCA) and L-FER Pidolate (Fe-PCA), and Pidolidone.

### Optional Organic Acid

The respiratory tract compositions of the present invention preferably comprise an optional organic acid that is suitable for use in combination with the optional pyroglutamic acid described hereinabove. It is believed that when the respiratory tract compositions comprise a combination of organic acid and Pyroglutamic acid, a composition is created that is more hostile to viruses known to contribute to cold and influenza-like symptoms.

The optional organic acid suitable for use herein can be included in the respiratory tract compositions as an individual organic acid or as a combination of organic acids, provided that the total organic acid concentration ranges from 0.01% to 10%, preferably from 0.05% to 5%, more preferably from 0.10% to 2.5%, by weight of the composition. The optional organic acid has a dissociation constant (pKa) of from 3.0 to 5.5. It has been shown that when an organic acid is combined with PCA, the respiratory tract compositions have an increased buffering capacity, providing a surface pH of the tissue treated in the nasal cavities or turbinates of from 3.0 to 5.5.

Nonlimiting examples of an optional organic acid suitable for use herein include ascorbic acid, monocarboxylic acids, dicarboxylic acids, tricarboxylic acids, and mixtures thereof. Specific nonlimiting examples of suitable monocarboxylic, dicarboxylic, or tricarboxylic acids include salicylic, fumaric, benzoic, glutaric, lactic, citric, malonic, acetic, glycolic, malic, maleic, adipic, succinic, aspartic, phthalic, tartaric glutamic, gluconic, ascorbic, and mixtures thereof. It has been found that these optional organic acids can be incorporated into the respiratory tract compositions of the present invention at the defined concentrations to create a hostile environment for viruses without significantly irritating specific areas of the respiratory tract such as the nasal tissues, notwithstanding that organic acids have been known to be potential irritants to respiratory tract tissues.

Although the optional organic acid has been shown to be effective when used in combination with pyroglutamic acid, it has been found that combinations of an organic acid and the ionic zinc compound are also effective in the prevention and treatment of cold and influenza-like symptoms. Combinations of an organic acid and ionic zinc compound can be included in the respiratory tract compositions with or without the addition of pyroglutamic acid to provide a therapeutic benefit in the prevention and treatment of cold and influenza-like symptoms

### Optional Mucoadhesive Polymer

The respiratory tract compositions of the present invention preferably comprise an optional mucoadhesive polymer that provides for improved retention of the compositions in areas of the respiratory tract such as the nasal cavity to result in improved prevention and treatment of cold and influenza-like symptoms without causing nasal irritation. It is known that mucoadhesive polymers can be incorporated into respiratory tract compositions such as nasal compositions to exhibit stimulus responsiveness. By stimulus responsiveness, it is meant that upon contacting the mucosal fluids or tissues, the compositions become sufficiently tacky or viscous to adhere to the tissues and do not quickly erode from the surface.

The optional mucoadhesive polymer suitable for use herein includes any solid or liquid used alone or in combination to impart a change in the viscosity of the respiratory tract compositions upon contact of the compositions with stimulus such as pH, body temperature, change in ionic concentration, and the like. Therefore, mucoadhesive polymers are sometimes commonly referred to as viscosity building polymers. It has been found that the incorporation of an optional mucoadhesive polymer that provides for a change in viscosity results in reducing and/or eliminating perceived irritation, especially perceived nasal irritation, that can be caused by the acidic nature of the respiratory tract compositions of the present invention.

The optional mucoadhesive polymer suitable for use herein provides for the adherence of the respiratory tract compositions to mucosal tissues, particularly nasal mucosal tissues, such that the acidic respiratory tract compositions comes into contact with mucosal tissues and fluids to result in a change in viscosity of the compositions in the respiratory tract area. As a result, the respiratory tract compositions of the present invention are maintained on the mucosal surface for periods longer than typical respiratory tract compositions, thus maintaining a virus-hostile environment for the improved prevention and treatment of cold and influenza-like symptoms. For example, if the respiratory tract compositions of the present invention contain an optional mucoadhesive polymer and are administered as a liquid respiratory tract composition, the composition is applied using an atomizing sprayer, and upon spraying into a respiratory tract area such as the nasal cavity the composition quickly forms a polymeric film, preferably a polymeric viscous film, that adheres to the nasal tissues. The polymeric film is preferably a thin polymer film, more preferably a thin polymeric viscous film, that is also resistant to erosion upon sneezing, blowing of the nose, or upon mucociliary clearance. The optional mucoadhesive polymer is also capable of changing the viscosity of the compositions in situ upon application of the respiratory tract compositions to the mucosal tissues and fluids.

The optional mucoadhesive polymer can be included in the respiratory tract compositions of the present invention as an individual mucoadhesive polymer or as a combination of mucoadhesive polymers, provided that the total concentration of mucoadhesive polymer ranges from 0.01% to 30%, preferably from 0.1% to 20%, more preferably from 1% to 5%, by weight of the composition.

The incorporation of the optional mucoadhesive polymer into the respiratory tract compositions of the present invention typically results in a composition that has a viscosity in the range of from 1 centipoise (cps) to 2000 cps, preferably from 1 cps to 1000 cps, more preferably from 5 cps to 500 cps, most preferably from 5 cps to 300 cps. The viscosity of the compositions can be measured by any known or otherwise effective technique employed to determine viscosity. Generally, the viscosity of the respiratory tract compositions of the present invention is determined using known methods such as those described in ASTM D1824-87, ASTM D1084-88, and ASTM D2196-86. Typical viscometers employed to measure viscosity include the Brookfield Syncho-Lectric Viscometer and the Haake Viscometer. For example, when the Brookfield Syncho-Lectric Viscometer is utilized for viscosity measurements, this viscometer is typically equipped with a spindle 4 to measure viscosities of less than 8,000 centipoise at low shear rates at given rotational speeds. Likewise, when the Haake Viscometer is utilized, a suitable Haake Viscometer is the Rheostress 1 model that is equipped with a probe (i.e., spindle) such as probe C35/2T wherein the viscosity measurement is performed over a temperature range of 5°C to 40°C at 50 revolutions per minute (rpm)/second (sec).

Known optional mucoadhesive polymers suitable fot use herein are selected from the group consisting of carboxypolymethylenes, carboxyvinyl polymers, homopolymers of acrylic acid crosslinked with an allyl ether of pentaerythritol, homopolymers of acrylic acid crosslinked with an allyl ether of sucrose, homopolymers of acrylic acid crosslinked with divinyl glycol, and mixtures thereof.

Nonlimiting examples of suitable homopolymers of acrylic acid crosslinked with an allyl ether of pentaerythritol or an allyl ether of sucrose are available from B. F. Goodrich Company under the tradename "Carbopol". Specific Carbopols include Carbopol 934, 940, 941, 956, 980, and mixtures thereof, Carbopol 980 is preferred among the optional carbopol mucoadhesive polymers. Polymers of this type have slightly acidic carboxyl group substituents. Such polymers generally have a pH of around 3 in water and are generally used by neutralization during preparation of compositions to form viscous films and/or gels. When the respiratory tract compositions of the present invention comprise one or more optional carpobol mucoadhesive polymers, generally these polymers are used at concentrations ranging from 0.01% to 2.5% by weight of the composition.

Nonlimiting examples of suitable homopolymers of acrylic acid crosslinked with divinyl glycol are available from B. F. Goodrich Company as polycarbophils under the tradename "Noveon."

Other nonlimiting examples of an optional mucoadhesive polymer suitable for use herein include natural polymers, polymeric cellulose derivatives, polyvinyl pyrrolidones (PVFs), dextran polymers, polyethylene oxide polymers including Polyox-600, thermoreversible polymers, ionic responsive polymers, copolymers of polymethyl vinyl ether and maleic anhydride, and mixtures thereof. Polymeric cellulose derivatives and thermoreversible polymers are preferred.

Specific nonlimiting examples of natural polymers suitable for use as an optional mucoadhesive polymer herein include arabic gums, tragacanth gums, agar polymers, xanthan gums, copolymers of alginic acid and sodium alginate, chitosan polymers, pectins, carageenans, pullulan polymers, modified starches, and mixtures thereof.

Specific nonlimiting examples of polymeric cellulose derivatives suitable for use as a preferred optional mucoadhesive polymer herein include hydroxy alkyl cellulose polymers including hydroxypropyl methylcellulose (HPMC) and hydroxypropyl cellulose (HPC), methyl cellulose polymers, carboxymethyl cellulose (CMC) polymers, salts of carboxymethyl cellulose including sodium salt of carboxymethyl cellulose, and mixtures thereof.

Specific nonlimiting examples of thermoreversible polymers suitable for use as a preferred optional mucoadhesive polymer herein include poloxamers including those poloxamers sold under the Lutrol F-127 and Lutrol F-68 tradenames, ethylhydroxy ethylcellulose (EHEC), and mixtures thereof.

Specific nonlimiting examples of ionic responsive polymers suitable for use as an optional mucoadhesive polymer herein include gelrite, gellan gum, Kelcogel F, and mixtures thereof.

Specific nonlimiting examples of copolymers of polymethyl vinyl ether and maleic anhydride suitable for use as an optional mucoadhesive polymer herein include such copolymers sold under the Gantrez tradename including Gantrez S and Gantrez MS type copolymers.

The optional mucoadhesive polymer suitable for use herein is more fully described in the Journal Pharmacy Pharmacology 53, pages 3-22, (2001 Edition); the International Journal of Pharmaceutics (1988, 1996 and 1998 Editions); and the Journal Controlled Release 62, pages 101-107, (1999 Edition); which descriptions are incorporated herein by reference.

### Optional pH Adjusting Agent

The respiratory tract compositions of the present invention can optionally comprise pH adjusting agents. Optional pH adjusting agents can be included in the respiratory tract compositions of the present invention to adjust the pH of the compositions to values less than 4.5. Therefore, when the compositions are applied to respiratory tract areas such as nasal tissues, the pH of the composition on the nasal tissues remains from 3.0 to 5.5, but is not so low as to cause irritation of the nasal tissues. Such optional pH adjusting agents include those normally associated with use in nasal compositions including compounds such as sodium bicarbonate, sodium phosphate, sodium hydroxide, ammonium hydroxide, triethanolamine, sodium citrate, disodium succinate, and mixtures thereof. If present, the optional pH adjusting agents are generally included at concentrations ranging from 0.01% to 5.0% by weight of the composition.

### Optional Chelating Agent

The respiratory tract compositions of the present invention can optionally comprise chelating agents which are believed to provide for enhanced antiviral activity. Optional chelating agents useful in the respiratory tract compositions of the present invention include those that chelate transition metal ions such as iron, copper, zinc and other such metals. Not to be bound by theory, it is reasonable to postulate that metal ions, specifically metal cations, play a major role in the formation of oxidizing species. Oxidizing reactions and free radical formation can contribute to cellular damage in inflammatory diseases. The optional chelating agents useful herein are known to dampen oxidation reactions. The optional chelating agents are stable and effective in non-aqueous and aqueous mediums, and at pH ranges between about 3 to about 6. Nonlimiting examples of suitable optional chelating agents include phytic acid, disodium and calcium salts of ethylene diamine tetraacetic acid (EDTA), tetrasodium EDTA, sodium hexametaphosphate (SHMP), di(hydroxyethyl)glycine, 8-hydroxyquinoline, and mixtures thereof.

The optional chelating agent can be included in the respiratory tract compositions in addition to the ionic zinc compound that provides for chelated zinc ions. Therefore, it is contemplated that when the respiratory tract compositions of the present invention comprise an optional cheating agent, the quantity of chelated zinc ions can increase. However, the ionic zinc compound providing chelated zinc ions provides for at least 50.1% chelated zinc ions whether the ionic zinc compound is incorporated into the respiratory tract compositions with or without an optional chelating agent.

If the respiratory tract compositions of the present invention comprise one or more optional chelating agents, the chelating agents are included at concentrations ranging from 0.001% to 10.00%, preferably from 0.005% to 5.0%, more preferably from 0.01% to 2%, by weight of the composition.

### Optional Preservatives

The respiratory tract compositions of the present invention can optionally comprise preservatives. Preservatives can optionally be included to prevent microbial contamination that can be attributed to dosing devices or the respiratory tract composition applied to the nose. Such optional preservatives include those normally associated with use in nasal compositions including benzalkonium chloride, chlorhexidine gluconate, phenyl ethyl alcohol, phenoxyethanol, benzyl alcohol, sorbic acid, thimerosal, phenylmercuric acetate, and mixtures thereof.

### Method of Manufacture

The respiratory tract compositions of the present invention may be prepared by any known or otherwise effective technique suitable for providing a pharmaceutical composition that provides a therapeutic benefit in the prevention and treatment of cold and influenza-like symptoms. The respiratory tract compositions are formulated to comprise the ionic zinc compounds described herein, wherein these compositions are then manufactured into final product forms of liquids, sprays, powders, irrigants, inhalants, pumps, drops, and so forth for administration to respiratory areas to prevent and treat symptoms due to respiratory tract viral infections.

When the respiratory tract compositions are administered using a pharmaceutically acceptable vehicle such as a liquid to deliver the compositions in product forms of sprays, pumps, droplets, and the like, the respiratory tract compositions are generally prepared by adding the ionic zinc compounds to a pre-mix solution of solubilized optional mucoadhesive polymer in a liquid vehicle such as water. While stirring, an optional pyroglutamic acid and optional organic acid can be added to the solution of ionic zinc compounds and optional mucoadhesive polymer. Next, a sensate mix is added while the solution is allowed to continue stirring. The sensate mix is typically added as a premix solution that can contain a combination of ingredients such as a combination of ethanol, menthol, peppermint oil, and spearmint oil. The pH of the resultant product should be between 3.0 and 5.5, however, a pH adjusting agent such as sodium hydroxide and/or disodium succinate can be added to maintain the pH of the resultant product to values between 3.0 to 5.5. These respiratory tract compositions administered in their liquid final product forms are especially suitable for incorporation into fill dropper vials, wherein the compositions are sprayed into a respiratory tract areas such as the nostrils or nasal turbinates for effective prevention and treatment of cold and influenza-like symptoms. Typically, about 100 microliters of the composition are sprayed into each nostril or nasal turbinate.

When the respiratory tract compositions of the present invention are administered using a pharmaceutically acceptable vehicle such as a powder, the compositions are generally prepared by dry blending the ionic zinc compounds, optional pyroglutamic acid, and an optional organic acid using a V-mixer. A pH adjusting agent such as sodium citrate can be added to the dry blend. The dry blend is then micronized using a fluid energy mill. The resultant micronized dry blend is then dry mixed with a powder filler such as lactose powder. This final powder respiratory tract composition can optionally be coated with a sensate premix using known spray coating technique. The final powder respiratory tract composition can be filled into a nasal inhalation metering pump to prevent and treat symptoms of the cold and influenza, wherein about 10 milligrams (mgs) of the final powder can be administered to a respiratory tract area such as a nostril or a turbinate.

As stated herein, the respiratory tract compositions of the present invention are suitable for administration in final product forms of liquids, sprays, pumps, inhalants, powders, and so forth. Suitable devices utilized in the administration of these final respiratory tract compositions include those commonly employed or otherwise effective liquid containers, droppers, spray containers including pressurized sprayers, pump containers, irrigation devices, inhalation devices, powder containers, atomizers, and so forth.

### EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention. All exemplified concentrations are weight-weight percents, unless otherwise specified.

Exemplary respiratory tract compositions of the present invention are exemplified in Table II hereinbelow. These respiratory tract compositions preferably comprise a sensate premix exemplified in Table I hereinbelow. The exemplified sensate premixes of Table I provide for respiratory tract compositions that are aesthetically pleasing in taste, flavor, coolness, smell, and the like.

The respiratory tract compositions exemplified hereinbelow in Table II are typically prepared by adding ionic zinc compounds to a pre-mix of solubilized optional mucoadhesive polymer in a liquid vehicle such as water. While stirring, pyroglutamic acid and/or an organic acid, if desired, are then added to the ionic zinc compounds and optional mucoadhesive polymer solution. Next, the sensate mix is added while the solution is allowed to continue stirring. The pH of the resultant product should be between 3.0 and 5.5, however, a pH adjusting agent such as sodium hydroxide and/or disodium succinate can be added to maintain the pH of the resultant product to values between 3.0 to 5.5. These final respiratory tract compositions are suitable for incorporation into fill dropper vials, wherein the compositions are sprayed into a respiratory tract areas such as the nostrils or turbinates for effective prevention and treatment of cold and influenza-like symptoms. Typically, about 100 microliters of the composition are sprayed into each nostril or turbinate. These compositions have at least about 50.1% of chelated zinc ions.

**Table I**

| **Component** | **Sensate Mix A** | **Sensate Mix B** | **Sensate Mix C** |
|---|---|---|---|
| | **(Wt. %)** | **(Wt. %)** | **(Wt. %)** |
| Ethanol | 47.16 | -- | -- |
| Menthol | 29.41 | 11.565 | 8.878 |
| Peppermint Oil | 17.61 | -- | -- |
| Spearmint Oil | 5.82 | -- | -- |
| Phenyl Ethyl Alcohol | -- | 77.495 | 83.281 |
| Camphor | -- | 6.971 | 4.998 |
| Eucalyptol | -- | 3.969 | 2.843 |
| **Total:** | **100** | **100** | **100** |

**Table II**

| **Component** | **Sample 1** | **Sample 2** | **Sample 3** | **Sample 4** | **Sample 5** |
|---|---|---|---|---|---|
| | **(Wt. %)** | **(Wt. %)** | **(Wt. %)** | **(Wt. %)** | **(Wt. %)** |
| Pyroglutamic Acid¹ | 0.35 | 0.70 | 1.00 | 0.37 | 1.70 |
| Succinic Acid² | 1.00 | 0.70 | 0.35 | 1.05 | 1.35 |
| Zinc Acetate Dihydrate³ | 0.12 | 0.012 | 0.12 | 0.12 | 0.12 |
| Zinc-EDTN⁴ | 0.255 | 0.255 | 0.255 | 0.255 | 0.255 |
| Polysorbate 80 | 0.05 | 0.05 | 0.05 | 0.12 | 0.08 |
| Carbopol 980⁵ | -- | -- | 1.20 | -- | -- |
| Hydroxypropyl methyl cellulose⁶ | 1.20 | -- | -- | 1.05 | 0.80 |
| Lutrol F-127⁷ | -- | 15.8 | -- | -- | -- |
| Sodiun saccharin | -- | 0.025 | 0.025 | 0.026 | -- |
| Sucralose | 0.025 | -- | -- | -- | 0.02 |
| Phenyl ethyl alcohol | 0.37 | 0.37 | 0.35 | -- | 0.25 |
| Sodium chloride | 0.20 | 0.20 | 0.50 | 0.21 | 0.20 |
| Sensate Mix A | 0.067 | -- | -- | 0.45 | -- |
| Sensate Mix B | -- | 0.49 | 0.45 | -- | 0.55 |
| Sodium hydroxide (30%) | -- | -- | 0.10 | -- | -- |
| Disodium succinate | 1.00 | 0.50 | -- | 0.46 | 0.25 |
| Propylene glycol | -- | -- | -- | 0.52 | 0.25 |
| Propyl gallate | -- | -- | -- | 0.01 | 0.02 |
| Deionized Water | q.s. 100 | q.s. 100 | q.s. 100 | q.s. 100 | q.s. 100 |

| | | | | | |
|---|---|---|---|---|---|
| Wt. % - weight percent 1 - pyroglutamic acid available from UCIB. France via Barnet Products Corp., New Jersey 2 - succinic acid available from DSM Fine Chemicals, UK 3 - zinc acetate dihydrate available from Verdugt B. V., Belgium 4 - zinc-EDTA available from Akzo Nobel Functional Chemicals by, The Netherlands 5 - Carbopol 980 available from B. F. Goodrich Company, USA. 6 - hydroxypropyl methylcellulose available from Colorcon Ltd, Kent, UK 7 - Lutrol F-127 available from BASP Speciality Chemicals, Mount Oliver, NJ, USA | | | | | |

## Claims

1. A respiratory tract composition comprising:
from 0.001% to 20% by weight of an ionic zinc compound providing from 0.1% to 49.9% of unchelated zinc ions by proportion of the total zinc ions; and
from 0.001% to 20% by weight of an ionic zinc compound providing from 50.1% to 99.9% of chelated zinc ions by proportion of the total zinc ions.

2. The respiratory tract composition of Claim 1 wherein the ionic zinc compound providing unchelated zinc ions is selected from zinc acetate, zinc sulphate, zinc chloride, zinc gluconate, zinc lactate, zinc ascorbate, and mixtures thereof.

3. The respiratory tract composition of Claim 1 or Claim 2 wherein the ionic zinc compound providing chelated zinc ions is selected from zinc citrate, zinc ethylenediaminetetraacetic acid disodium salt (zinc-EDTA), zinc succinate, zinc tartrate, zinc malate, and mixtures thereof.

4. The respiratory tract composition of any preceding claim wherein the composition further comprises from 40% to 99.98% by weight of a pharmaceutically acceptable vehicle selected from water, ethanol, propylene glycol, polyethylene glycol, transcutol, glycerol, a liquid aerosol propellant, and mixtures thereof.

5. The respiratory tract composition of Claim 4 wherein the composition further comprises from 0.01% to 20% by weight of pyroglutamic acid.

6. The respiratory tract composition of Claim 4 wherein the composition further comprises from 0.01% to 10% by weight of an organic acid selected from ascorbic acid, salicylic acid, fumaric acid, benzoic acid, glutaric acid, lactic acid, citric acid, malonic acid, acetic acid, glycolic acid, malic acid, maleic acid, adipic acid, succinic acid, aspartic acid, phthalic acid, tartaric acid, glutamic acid, gluconic acid, ascorbic acid, and mixtures thereof.

7. The respiratory tract composition of Claim 4 wherein the composition further comprises from 0.01% to 30% by weight of a mucoadhesive polymer selected from carboxypolymethylenes, carboxyvinyl polymers, homopolymers of acrylic acid crosslinked with an allyl ether of pentaerythritol, homopolymers of acrylic acid crosslinked with an allyl ether of sucrose, homopolymers of acrylic acid crosslinked with divinyl glycol, natural polymers, polymeric cellulose derivatives, polyvinyl pyrrolidones (PVPs), dextran polymers, polyethylene oxide polymers, thermoreversible polymers, ionic responsive polymers, copolymers of polymethyl vinyl ether and maleic anhydride, and mixtures thereof.

8. The respiratory tract composition of Claim 5 wherein the composition has a pH in the range of from 3.0 to 5.5; and further comprises
a pH adjusting agent selected from sodium bicarbonate, sodium phosphate, sodium hydroxide, ammonium hydroxide, triethanolamine, sodium citrate, disodium succinate, and mixtures thereof.

9. The respiratory tract composition of any preceding claim wherein the composition is a nasal composition selected from nasal liquids, nasal sprays, nasal inhalants, nasal irrigants, nasal powders, nasal drops, and mixtures thereof.

10. The use of an ionic zinc compound in the manufacture of a respiratory tract composition according to any preceding claim for treating symptoms of colds or influenza by administration of the composition to mucosal tissue or fluid of the respiratory tract.

## Patentansprüche

1. Zusammensetzung für den Atemtrakt, umfassend:
zu 0,001 Gew.-% bis 20 Gew.-% eine ionische Zinkverbindung, die, bezogen auf die gesamten Zinkionen, 0,1 % bis 49,9 % unchelierte Zinkionen bereitstellt; und
zu 0,001 Gew.-% bis 20 Gew.-% eine ionische Zinkverbindung, bezogen auf die gesamten Zinkionen 50,1 % bis 99,9 % chelierte Zinkionen.

2. Zusammensetzung für den Atemtrakt nach Anspruch 1, wobei die ionische Zinkverbindung, die unchelierte Zinkionen bereitstellt, ausgewählt ist aus Zinkacetat, Zinksulfat, Zinkchlorid, Zinkgluconat, Zinklactat, Zinkascorbat und Mischungen davon.

3. Zusammensetzung für den Atemtrakt nach Anspruch 1 oder Anspruch 2, wobei die ionische Zinkverbindung, die chelierte Zinkionen bereitstellt, ausgewählt ist aus Zinkcitrat, Zinkethylendiamintetraessigsäure-Dinatriumsalz (Zink-EDTA), Zinksuccinat, Zinktartrat, Zinkmalat und Mischungen davon.

4. Zusammensetzung für den Atemtrakt nach einem der vorstehenden Ansprüche, wobei die Zusammensetzung ferner zu ungefähr 40 Gew.-% bis ungefähr 99,98 Gew.-% einen pharmazeutisch unbedenklichen Träger, ausgewählt aus Wasser, Ethanol, Propylenglycol, Polyethylenglycol, Transcutol, Glycerol, einem flüssigen Aerosol-Treibmittel und Mischungen davon, umfasst.

5. Zusammensetzung für den Atemtrakt nach Anspruch 4, wobei die Zusammensetzung ferner zu 0,01 Gew.-% bis 20 Gew.-% Pyroglutaminsäure umfasst.

6. Zusammensetzung für den Atemtrakt nach Anspruch 4, wobei die Zusammensetzung ferner zu 0,01 Gew.-% bis 10 Gew.-% eine organische Säure, ausgewählt aus Ascorbinsäure, Salicylsäure, Fumarsäure, Benzoesäure, Glutarsäure, Milchsäure, Citronensäure, Malonsäure, Essigsäure, Glycolsäure, Äpfelsäure, Maleinsäure, Adipinsäure, Bernsteinsäure, Asparaginsäure, Phthalsäure, Weinsäure, Glutaminsäure, Gluconsäure, Ascorbinsäure und Mischungen davon, umfasst.

7. Zusammensetzung für den Atemtrakt nach Anspruch 4, wobei die Zusammensetzung ferner zu 0,01 Gew.-% bis 30 Gew.-% ein mukoadhäsives Polymer, ausgewählt aus Carboxypolymethylenen, Carboxyvinylpolymeren, Homopolymeren von Acrylsäure, vernetzt mit einem Allylether von Pentaerythrit, Homopolymeren von Acrylsäure, vernetzt mit einem Allylether von Saccharose, Homopolymeren von Acrylsäure, vernetzt mit Divinylglycol, natürlichen Polymeren, polymeren Cellulosederivaten, Polyvinylpyrrolidonen (PVP), Dextranpolymeren, Polyethylenoxidpolymeren, thermoreversiblen Polymeren, ionischen reagierenden Polymeren, Copolymeren von Polymethylvinylether und Maleinsäureanhydrid und Mischungen davon, umfasst.

8. Zusammensetzung für den Atemtrakt nach Anspruch 5, wobei die Zusammensetzung einen pH im Bereich von 3,0 bis 5,5 hat und ferner einen pH-Regler, ausgewählt aus Natriumhydrogencarbonat, Natriumphosphat, Natriumhydroxid, Ammoniumhydroxid, Triethanolamin, Natriumcitrat, Dinatriumsuccinat und Mischungen davon, umfasst.

9. Zusammensetzung für den Atemtrakt nach einem der vorstehenden Ansprüche, wobei die Nasalzusammensetzung aus Nasalflüssigkeiten, Nasensprays, Naseninhalationsmitteln, Nasenspülungen, Nasenpulvern, Nasentropfen und Mischungen davon ausgewählt ist.

10. Verwendung einer ionischen Zinkverbindung bei der Herstellung einer Zusammensetzung für den Atemtrakt nach einem der vorstehenden Ansprüche zur Behandlung der Symptome von Erkältungen oder Grippe durch Verabreichung der Zusammensetzung an Schleimhautgewebe oder -flüssigkeit des Atemtrakts.

## Revendications

1. Composition pour système respiratoire comprenant :
de 0,001 % à 20 % en poids d'un composé de zinc ionique fournissant de 0,1 % à 49,9 % d'ions zinc non chélatés par rapport aux ions zinc totaux ; et
de 0,001 % à 20 % en poids d'un composé de zinc ionique fournissant de 50,1 % à 99,9 % d'ions zinc chélatés par rapport aux ions zinc totaux.

2. Composition pour système respiratoire selon la revendication 1, dans laquelle le composé de zinc ionique fournissant des ions zinc non chélatés est choisi dans le groupe constitué d'acétate de zinc, sulfate de zinc, chlorure de zinc, gluconate de zinc, lactate de zinc, ascorbate de zinc, et leurs mélanges.

3. Composition pour système respiratoire selon la revendication 1 ou la revendication 2, dans laquelle le composé de zinc ionique fournissant des ions zinc chélatés est choisi dans le groupe constitué de citrate de zinc, sel disodique d'acide éthylènediaminetétra-acétique (zinc-EDTA), succinate de zinc, tartrate de zinc, malate de zinc, et leurs mélanges.

4. Composition pour système respiratoire selon l'une quelconque des revendications précédentes, où la composition comprend, en outre, de 40 % à 99,98 % en poids d'un véhicule pharmaceutiquement acceptable choisi parmi l'eau, l'éthanol, le propylène glycol, le polyéthylène glycol, le transcutol, le glycérol, un propulseur aérosol liquide, et leurs mélanges.

5. Composition pour système respiratoire selon la revendication 4, où la composition comprend, en outre, de 0,01 % à 20 % en poids d'acide pyroglutamique.

6. Composition pour système respiratoire selon la revendication 4, où la composition comprend, en outre, de 0,01 % à 10 % en poids d'un acide organique choisi parmi l'acide ascorbique, l'acide salicylique, l'acide fumarique, l'acide benzoïque, l'acide glutarique, l'acide lactique, l'acide citrique, l'acide malonique, l'acide acétique, l'acide glycolique, l'acide malique, l'acide maléique, l'acide adipique, l'acide succinique, l'acide aspartique, l'acide phtalique, l'acide tartrique, l'acide glutamique, l'acide gluconique, l'acide ascorbique, et leurs mélanges.

7. Composition pour système respiratoire selon la revendication 4, où la composition comprend, en outre, de 0,01 % à 30 % en poids d'un polymère muco-adhésif choisi parmi les carboxypolyméthylènes, les polymères carboxyvinyle, des homopolymères d'acide acrylique réticulés avec un éther d'allyle de penta-érythritol, des homopolymères d'acide acrylique réticulés avec un éther d'allyle de saccharose, des homopolymères d'acide acrylique réticulés avec du divinyl glycol, des polymères naturels, des dérivés de cellulose polymères, des polyvinylpyrrolidones (PVP), des polymères de dextrane, des polymères d'oxyde de polyéthylène, des polymères thermo-réversibles, des polymères ioniques répondants, des copolymères de polyméthyl-vinyl-éther et d'anhydride maléique, et leurs mélanges.

8. Composition pour système respiratoire selon la revendication 5, où la composition a un pH dans la gamme de 3,0 à 5,5 ; et comprend, en outre, un agent d'ajustement du pH choisi parmi le bicarbonate de sodium, le phosphate de sodium, l'hydroxyde de sodium, l'hydroxyde d'ammonium, la triéthanolamine, le citrate de sodium, le succinate de disodium, et leurs mélanges.

9. Composition pour système respiratoire selon l'une quelconque des revendications précédentes, où la composition est une composition nasale choisie parmi les liquides nasaux, les aérosols nasaux, les inhalateurs nasaux, les irrigants nasaux, les poudres nasales, les gouttes nasales, et leurs mélanges.

10. Utilisation d'un composé de zinc ionique dans la fabrication d'une composition pour système respiratoire selon l'une quelconque des revendications précédentes, pour le traitement des symptômes de rhumes ou de grippe par l'administration de la composition sur un tissu muqueux ou un fluide du système respiratoire.
